# EUROPEAN PATENT APPLICATION

(11) **EP 1 302 214 A2**
(43) Date of publication of application: **16.04.2003**
(21) Application number: 02257004.8
(22) Date of filing: 09.10.2002
(51) Int. Cl.: A61N 1/05

(54) **System and device for placing a body implantable lead in the coronary sinus region of the heart**

(30) Priority: 16.10.2001 US 981958
(71) Applicant: PACESETTER, INC., Sylmar, CA 91392-9221 (US)
(72) Inventor: Florio, Joseph J., La Canada, CA 91011 (US); Bornzin, Gene A., Simi Valley, CA 93063 (US); Morgan, Kevin L., Simi Valley, CA 93063 (US); Williams, Sheldon, Green Valley, CA 91350 (US)
(74) Representative: Rees, David Christopher

(57) **Abstract**

An implantable stimulation lead system comprises a lead including a lead body dimensioned for placement inside the coronary sinus region. The lead system further comprises a device (60) dimensioned for insertion within a lumen in the lead, the device (60) including a main body (62); a steering knob (68) secured to a proximal extremity (64) of the main body (62); and a flexible distal portion (70) secured to a distal extremity (66) of the main body (62). The main body (62) has a length such that, with the main body (62) of the device substantially completely advanced within the lead, the flexible distal portion (70) of the device projects distally from the aperture in the distal tip of the lead body. The flexible distal portion (70) of the device may comprise a proximal section and a distal section, the distal section being more flexible, and thus softer, than the proximal section.

## Description

The present invention relates generally to body implantable leads such as endocardial leads for stimulating selected tissue within the heart, and more particularly, to a system, device and method for delivering and steering the distal portions of such leads into position relative to the body tissue to be stimulated and specifically the coronary sinus region of the heart.

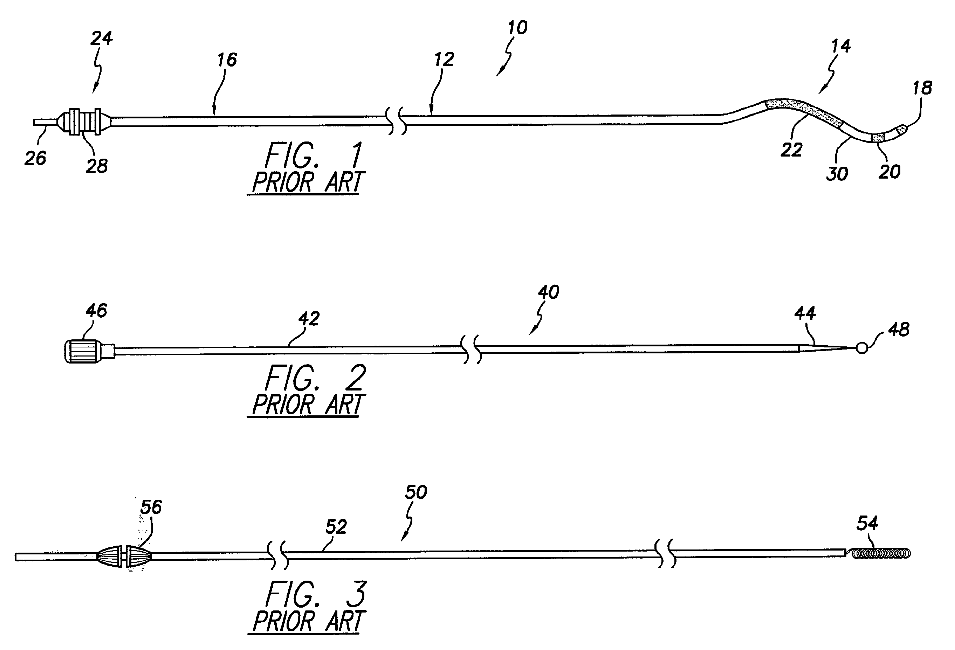

FIG. 1 shows an example of a typical known body implantable lead 10 including a lead body 12 having a distal portion 14 and a proximal portion 16. The distal portion 14 may carry a variety of combinations of pacing, sensing and/or defibrillation electrodes, all well known in the art. In the specific example shown in FIG. 1, the distal portion of the lead assembly includes a tip electrode 18 at the distal extremity of the lead body for delivering electrical stimuli to selected tissue of the heart, a sensor electrode 20 in the form of a ring electrode positioned proximally of the tip electrode 18 and a shocking or defibrillation electrode 22 disposed proximally of the sensor electrode 20. These electrodes are electrically connected to a connector assembly 24 at the proximal extremity of the lead assembly. The connector assembly 24 includes a hollow or tubular connector pin 26 and plurality of contact rings 28. Further, as is well known in the art, the connector assembly 24 may comprise a bifurcated or trifurcated structure depending upon the number and functions of the electrodes carried by the distal portion 14 of the lead assembly. A lead assembly such as that shown in FIG. 1, designed for implantation in the coronary sinus region of the heart, will typically include an anchoring or fixation means 30 including, in the example under consideration, a sinuous configuration of the distal portion of the lead. As is known, with such a configuration the bend or bends in the distal portion of the lead press against the walls of the coronary vessel within which the distal portion of the lead is implanted and the friction caused by such biasing facilitates the anchoring or fixation of the distal portion of the lead.

Presently, the distal portions of body implantable leads such as that shown in FIG. 1 are maneuvered into position relative to the tissue to be stimulated by means of a stylet and/or a guide wire. The use of such implantation expedients is well known in the art. Thus, for example, a stylet may be passed through the hollow electrical connector pin 26 at the proximal end of the lead assembly 10 into and along a central cavity or lumen of a coiled electrical conductor or a central lumen otherwise formed in the lead body 12 to enable the implanting physician to orient the distal portion 14 of the lead assembly and to position the electrodes under fluoroscopy at a desired location within the heart. To reduce frictional resistance to advancement of the stylet within the lead body, the lumen may include a thin wall PTFE tube through which the stylet is passed. The distal extremity of the stylet typically engages a plug or other stop surface within the distal portion of the lead assembly. The stylet may comprise a steerable assembly so that a desired curvature in the distal end portion of the lead may be imparted during the introduction of the lead assembly 10 to guide it through curvatures in the patient's vascular system.

FIG. 2 is a longitudinal side view of a typical stylet 40 in use today for advancing an endocardial lead within a patient's venous system and, for left side pacing, placing the distal portion and the electrodes carried thereby within the coronary sinus region of the heart. The stylet 40 includes a main body 42 having a tapered portion 44 on the distal end of the main body and a steering knob 46 secured (usually permanently) to the proximal extremity of the main body. The knob 46 not only aids in the maneuvering and handling of the stylet but is often also used to identify the stylet. The tapered portion 44 of the main body terminates at its distal extremity in an enlarged, typically spherical tip 48 which helps prevent perforation of the lead insulation layer and provides an enlarged area for engaging a plug or stop within the distal portion of the lead. The tapered portion 44 is relatively flexible and aids in maneuvering and steering the lead during implantation into difficult-to-reach heart regions. The main body 42 of the stylet typically comprises a relatively stiff wire of stainless steel or the like so as to facilitate the advancement of the stylet in the venous system and provide the stylet with a high degree of trackability. The main body 42, including the tapered portion 44 thereof, is typically a one piece structure. The length of the main body 42 depends on the particular implantation configuration and the length of the associated lead. Typically, a stylet is a few centimeters longer than the lead assembly that is intended to be placed thereby. By way of example, a lead assembly such as that shown in FIG. 1, having a lead body length ranging, for example, from 75 to 90cm, will be implanted using a stylet having a main body about 80 to 105cm long, that is, about 5 to 15cm longer than the associated lead body.

The implantation of a lead in the coronary sinus region is often difficult because of the extreme curvatures in the veins, their narrowness, anomalies in the vascular anatomy because of disease, and the number of veins which may communicate with the desired lead feed path. Stylets are often found to be too inflexible to be steered within the tortuous vasculature of the coronary sinus region. Thus, a more common approach to the left side implantation of an intravenous lead is the use of a flexible guide wire over which the lead is slid to its destination. For this purpose, the lead is provided with a tip electrode having a central aperture through which the guide wire can pass.

With reference to FIG. 3, there is shown a typical guide wire 50 in present use. The guide wire 50 comprises a main wire body 52 and a flexible end 54 comprising a finely coiled structure welded to the distal extremity of the main body 52. In comparison to a stylet, the guide wire 50 is relatively long, for example, 160 to 180cm in overall length or about twice as long as the lead that is to be implanted. A guide wire of such length is necessary because after the guide wire has been advanced into position within the target coronary vein, the proximal portion of the guide wire projecting from the introduction point must be long enough to receive the lead. The guide wire is steered using a releasable or selectively lockable clamp 56 which, when loosened, can be slid along the main body 52 of the guide wire. Such a slidable, selectively lockable clamp is necessary because of the substantial length of the guide wire; the clamp is slid as required along the wire and locked in place at a position where it can be easily reached by the implanting physician during the implantation procedure.

In use, the guide wire 50 is inserted into the patient's vascular system utilizing an introducer. After the guide wire is properly placed, the introducer is removed, the clamp 56 is removed and the lead is then slid over the exposed part of the guide wire and advanced "over-the-wire" into the vessel. The clamp 56 is then slid back on the guide wire and tightened. These are awkward steps since the proximal extremity of the guide wire is typically a long distance from the introduction point. The flexible end 54 of the guide wire is maneuvered into the target vessel using the releasable clamp 56. After the lead is advanced over the wire into the vessel, and is in place therein, the guide wire is withdrawn. This, also, is an awkward maneuver given the length of the guide wire.

In accordance with one aspect of the present invention, there is provided an implantable stimulation lead system, a preferred embodiment of which comprises a lead including a lead body dimensioned for placement inside the coronary sinus region. The lead body has at least one electrode positioned at a distal end of the lead body, the distal end of the lead body including a distal tip. The lead further includes a lumen extending the length of the lead and communicating with an aperture in the distal tip. The lead system further comprises a device dimensioned for insertion within the lumen, the device including a main body; a steering knob secured to a proximal extremity of the main body; and a flexible distal portion secured to a distal extremity of the main body. The main body has a length such that, with the main body of the device substantially completely advanced within the lead, the flexible distal portion of the device projects distally from the aperture in the distal tip of the lead body.

Pursuant to other forms of the lead system of the invention, the main body of the device is formed of wire, and the flexible distal portion of the device comprises a wire coil.

In accordance with another exemplary form of the lead system of the invention, the flexible distal portion of the device comprises a proximal section and a distal section, the distal section being more flexible, and thus softer, than the proximal section. Still further, the proximal section and the distal section of the flexible distal portion of the device comprise wire coils.

Pursuant to another embodiment of the lead system of the invention, the said proximal and distal sections comprise a unitary structure. In one form of this embodiment, the proximal and distal sections are cylindrical in shape, with the proximal section having an outer diameter smaller than that of the main body and the distal section having an outer diameter smaller than that of the proximal section. In another form of this embodiment, the proximal section is cylindrical and has a diameter smaller than that of the main body, and the distal section comprises a thin leaf. Preferably, the thin leaf has a rectangular shape, with a width equal to the diameter of the proximal section. Further, the flexible distal portion of the device may include a wire coil surrounding the proximal and distal sections of the flexible distal portion. Further in this connection, the thin leaf includes a distal tip and the wire coil surrounding the proximal and distal sections of the flexible distal portion has an end attached to the distal tip of the thin leaf and another end attached to the distal extremity of the main body.

In accordance with another aspect of the present invention, there is provided a device for delivering a body implantable lead, a preferred embodiment of which device comprises a main wire body having a proximal extremity and a distal extremity; a steering knob secured to the proximal extremity of the main wire body; and a flexible distal portion having a proximal end secured to the distal extremity of the main body, the flexible distal portion comprising a wire coil. The wire coil forming the flexible distal portion of the device may include a proximal section and a distal section, the distal section being more flexible than the proximal section. Further, the proximal section and the distal section of the flexible distal portion of the device may comprise wire coils. Alternatively, the proximal and distal sections may comprise a unitary structure. In one form of this alternative, the proximal section is cylindrical and has a diameter smaller than that of the main body, and the distal section comprises a thin leaf. Still further, the thin leaf preferably has a rectangular shape with a width substantially the same as the diameter of the proximal section. Further yet, the flexible distal portion of the device preferably includes a wire coil surrounding the proximal and distal sections of the flexible distal portion. More specifically in this connection, the thin leaf includes a distal tip and the wire coil surrounding the proximal and distal sections of the flexible distal portion has one end attached to the distal tip of the thin leaf and another end attached to the proximal end of the flexible distal portion.

In accordance with yet another aspect of the invention, there is provided a method of implanting an electrode of an endocardial lead at an implantation site within a cardiac vein accessible via the superior vena cava (SVC), coronary os and the coronary sinus region, the lead including a distal portion and a lumen communicating with an aperture in a tip electrode, said implanting being effected using a device comprising a main body, a steering knob secured to a proximal extremity of the main body and a flexible distal portion affixed to and extending distally from a distal extremity of the main body, the method comprising the steps of inserting the device into the lumen of the lead with the flexible distal portion substantially entirely contained within the distal portion of the lead; feeding an introducer sheath along a predetermined path including at least the SVC and coronary os; inserting the distal portion of the lead into the introducer sheath and advancing the lead until the distal portion of lead reaches the coronary sinus region; advancing the device relative to the lead to extend the flexible distal portion of the device from the aperture in the tip electrode; manipulating the steering knob on the proximal end of the main body of the device as necessary to maneuver the flexible distal portion of the device into said implantation site; slidably advancing the lead over the device to move said electrode into place at the implantation site; retracting the device and removing the device from the lead; and retracting and removing the introducer sheath.

It will be seen that unlike the prior art, the lead body provides the guide or delivery tool for the flexible distal portion of the device of the invention. It will also be appreciated that the device of the present invention has the advantage of greatly speeding up the process of lead implantation. It avoids both the relative inflexibility of a stylet and the awkwardness of using an unduly long guide wire yet it is designed for use with a lead body designed for over-the-wire placement with all of the attendant advantages.

The invention may be carried into practice in various ways and some embodiments will now be described by way of example with reference to the accompanying drawings, in which:
FIG. 1 is a side view of a typical endocardial lead assembly for placement in a vessel in the coronary sinus region of the heart;
FIG. 2 is a side view of a stylet in accordance with the prior art;
FIG. 3 is a side view of a guide wire in accordance with the prior art;
FIG. 4 is a side view of a device in accordance with a first embodiment of the present invention for placing a body implantable lead in the coronary sinus region of the heart;
FIG. 5 is a side view of a device in accordance with a second embodiment of the present invention;
FIG. 6 is a side view of the distal portion of a device in accordance with a third embodiment of the present invention;
FIG. 7 is a side view of the distal portion of a device in accordance with a fourth embodiment of the present invention;
FIG. 8 is a perspective view of the distal portion of a device in accordance with a fifth embodiment of the present invention;
FIG. 9 is a side view, partly in cross section, of the distal portion shown in FIG. 8;
FIG. 10 is a top view, partly in cross section, of the distal portion shown in FIG. 8;
FIG. 11 is a side view of the distal portion of a device in accordance with a sixth embodiment of the present invention;
FIG. 12 is a side view of an endocardial lead with the device of the invention in place therein preparatory to implantation of the lead; and
FIGS. 13-15 are perspective views of the anterior portion of the heart showing steps in the use of the device of the present invention.

It will be appreciated that for the sake of clarity, the dimensions and proportions of the devices shown in the drawings have been greatly exaggerated.

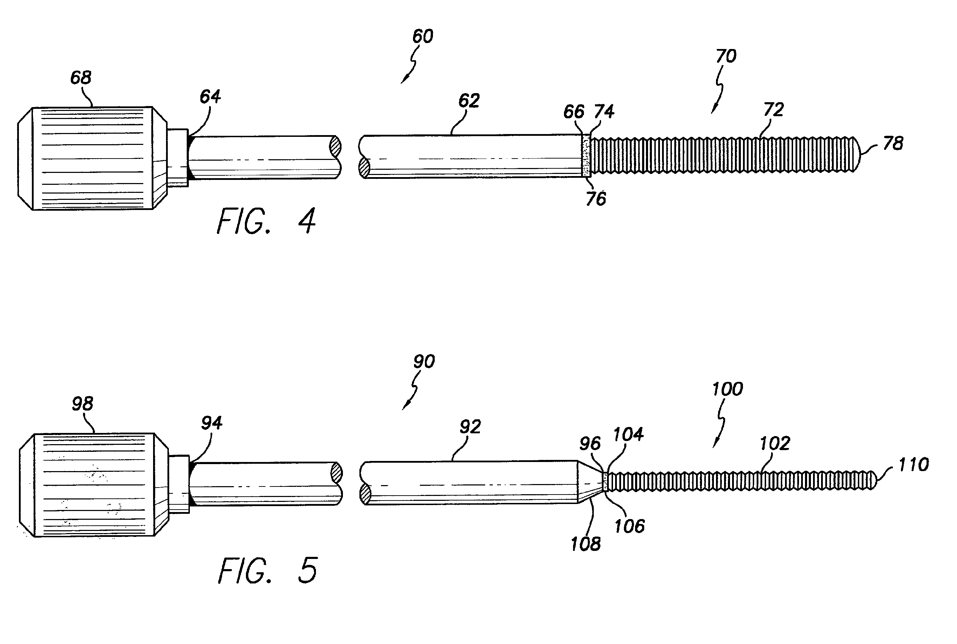

FIG. 4 shows a device 60, comprising a first embodiment of the invention, for directing and steering a body implantable lead, such as the lead body 12 of the lead assembly 10, into contact with heart tissue to be stimulated. Although the device 60 may be used to position a body implantable lead in the right chambers of the heart, that is, the right atrium and/or right ventricle, the device 60 has particular utility for placing a lead transvenously within the coronary sinus region of the heart to perform pacing, sensing and/or defibrillation of the left atrium and/or left ventricle. As used herein, the phrase "coronary sinus region" refers to the coronary sinus, great cardiac vein, left marginal vein, left posterior ventricular vein, middle cardiac vein, and/or small cardiac vein or any other cardiac vein accessible via the coronary sinus.

The device 60 includes a relatively stiff main wire body 62 of stainless steel or like biocompatible, biostable material. By way of example and not limitation, the wire body 62 may have a diameter of .012 to .018 inch, preferably about .014 inch. The main body of the device includes a proximal extremity 64 and a distal extremity 66.

The device 60 further comprises a steering knob 68 which, like the steering knob 46 on the prior art stylet 40 (FIG. 2) and unlike the releasable clamp 56 on the prior art guide wire 50 (FIG. 3), is secured, preferably permanently, to the proximal extremity 64 of the main wire body 62. The device 60 also includes a distal portion 70 comprising a wire coil 72 having a proximal end 74 affixed to the distal extremity 66 of the main wire body 62. In the embodiment of FIG. 4, the wire coil 72 has an outer diameter substantially the same as that of the main wire body 62 so as to form an isodiametric structure. The wire coil 72 is preferably made of stainless steel or a similar biocompatible, biostable alloy, in which case it is affixed to the distal extremity 66 of the main wire body 62 by means of a brazed joint or weld 76.

The wire coil 72 comprising the distal portion 70 of the device 60 may be best described as a flexible or "floppy" element. By way of example and not limitation, this attribute may be provided by forming the wire coil 72 of No. 44 to 36 Brown and Sharpe gauge wire (.00198 to .005 inch diameter, respectively) wound with a pitch of about 505 to about 200 turns per inch, respectively. The wire coil 72 has a distal tip 78 that may be melted or fused to form a smooth leading surface. The steering knob 68 secured to the proximal extremity of the main wire body 62 is used by the implanting physician to manipulate the main wire body 62 so as to direct and steer the distal portion 70 of the device 60 to a destination within the cardiac sinus region, as will be described below.

Like the main body 42 of the stylet 40 shown in FIG. 2, the overall length of the main body 62 and distal portion 70 of the device 60 may be about 80 to 105cm, by way of example and not limitation. Thus, like the stylet of the prior art, the device 60 of the present invention is slightly longer than the typical lead to be implanted with the aid of the device 60. The distal portion 70 may be of various lengths, for example, about 5-15cm.

Although variations will suggest themselves to skilled artisans, the basic relationship between the overall length of the main body 62 and the distal portion 70 of the device 60 and the overall length of the lead 10 (from the connector assembly to the tip aperture) is such that with the main body substantially fully advanced into the lead, the flexible distal portion 70 projects distally from the aperture in the lead tip.

FIG. 5 shows a device 90 in accordance with a second embodiment of the present invention. The device 90 of the second embodiment is identical to the first embodiment shown in FIG. 4 to the extent that it includes, like the device 60, a main wire body 92 having proximal and distal extremities 94 and 96, respectively, a steering knob 98 secured to the proximal extremity 94 of the main wire body 92, and a distal portion 100. Like the first embodiment, the distal portion 100 of the device 90 comprises a wire coil 102 having a proximal end 104 affixed to the distal extremity 96 of the main wire body 92. In the case of the second embodiment, however, the outer diameter of the wire coil 102 is less than that of the main wire body 92 in order to provide the wire coil 102 with even greater flexibility. The wire coil 102 is again preferably made of a biocompatible, biostable alloy such as stainless steel and it is secured to the distal extremity 96 of the main wire body 92 by means of a weld 106 or similar bond. The main wire body 92 includes a taper 108 at the distal end joining the larger diameter main wire body with the smaller diameter distal extremity 96. Again, the wire coil 102 includes a distal tip 110 that may be melted or fused to form a smooth leading surface. The overall length of the main wire body 92 and distal portion 100 as well as the materials may be as described in connection with the first embodiment.

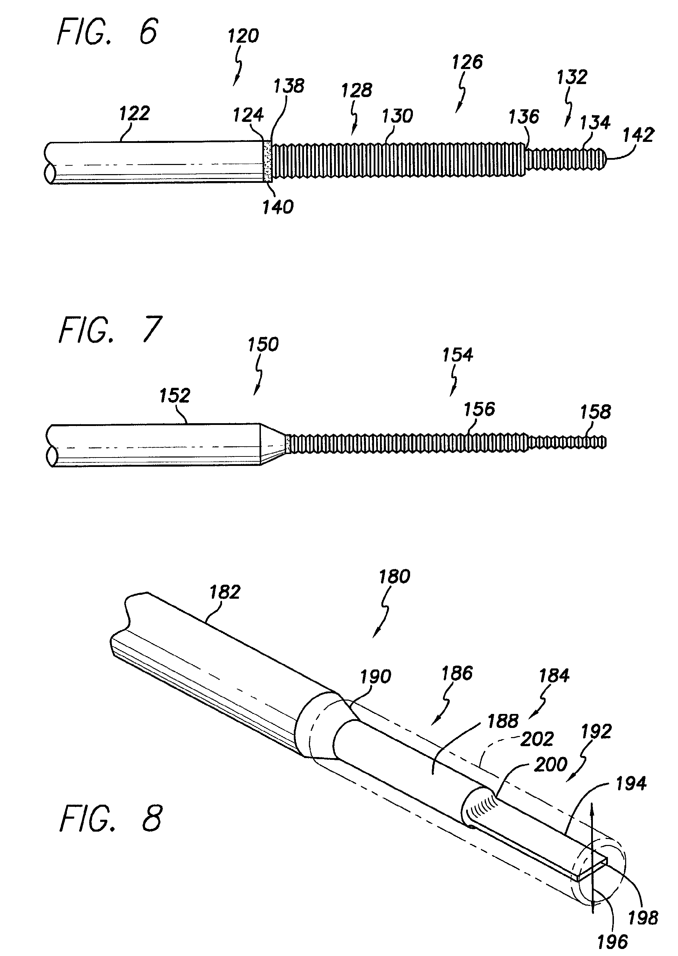

FIG. 6 shows the distal part of a device 120 in accordance with a third embodiment of the present invention. The device 120 includes a main body 122 having a distal extremity 124 and a steering knob (not shown) secured to a proximal extremity, along the lines of that already described in connection with the previous embodiments. The device 120 includes a flexible or floppy distal portion 126 comprising a proximal section 128 formed of a wire coil 130 having an outer diameter substantially the same as that of the main wire body 122 and a distal section 132 comprising a very soft wire coil 134 having a diameter smaller than that of the proximal section 128. The wire coils 130 and 134 comprising the proximal and distal sections of the distal portion 126 of the device may be formed of a continuous wire wound about a stepped mandrel to define the two sections of the distal portion 126. Alternatively, the wire coil sections 130 and 134 may be separately formed and joined, by brazing or welding, at an interface 136 between the sections. By way of example and not limitation, the wire of the wire coil section 130 may have a gauge and coil pitch as described above in connection with the embodiment of FIG. 4. Further, by way of example and not limitation, the small wire coil 134 may be formed of No. 45 to 38 Brown and Sharpe gauge wire (.00176 to .00396 inch diameter, respectively) with a pitch of about 560 to about 250 turns per inch, respectively. As before, the distal portion 126 has a proximal end 138 affixed to the distal extremity 124 by means of a weld 140 or the like. The proximal section 128 of the distal portion 126 of the device 120 is very flexible; the distal section 132, given its smaller diameter, is even more flexible and softer and this construction facilitates the feeding of the distal portion 126 of the device through the tortuous vessels in the coronary sinus region of the heart without damage to the vessel walls. The smaller diameter wire coil 134 forming the distal section has a distal tip 142 that may be melted or fused to form a smooth surface.

FIG. 7 shows the distal part of a device 150 in accordance with a fourth embodiment of the invention. The device 150 includes a main wire body 152 and a distal portion 154 comprising a proximal wire coil section 156 and a distal wire coil section 158, similar to that shown in FIG. 6. The diameter of the proximal wire coil section 156 in FIG. 7 is smaller than the outer diameter of the main wire body 152 and the distal wire coil section 158 has a diameter that is smaller than that of the proximal wire coil section 156. As in the case of the second embodiment shown in FIG. 5, the object is to provide the distal portion 154 of the device 150 with even greater flexibility and softness than that obtainable from the embodiment of FIG. 6.

FIGS. 8-10 show the distal part of a device 180 in accordance with a fifth embodiment of the invention. The device 180 includes a main wire body 182 and a flexible distal portion 184 that, in accordance with a preferred form of the fifth embodiment, comprises an extension of the main wire body 182. The distal portion 184 comprises a proximal section 186, having an outer cylindrical surface 188 concentric with that of the main wire body 182 and joined thereto by means of a tapered transition 190. The distal portion 184 further includes a distal section 192 comprising a distally projecting, thin, very flexible ribbon or flat leaf 194 that is easily twisted and is particularly pliant or bendable along an axis 196 perpendicular to the broad surfaces of the leaf 194. The leaf 194 has a distal tip 198 and, in accordance with the specific embodiment under consideration, the leaf preferably has a width equal to the diameter of the proximal section 186 of the distal portion and is joined thereto by means of a tapered transition 200. Although the distal portion 184 of the device and the main wire body 182 may be a one piece or unitary structure, it will be evident that the main body 182 and the proximal and distal sections 186 and 192 may be constructed of separate pieces welded or otherwise bonded together. The proximal section 186, given its smaller diameter, is considerably more flexible than the main wire body 182 and, in turn, the leaf 194 is even more flexible and particularly along the axis 196. The distal portion 184 of the device includes a wire coil 202 wound around the distal portion 184 and affixed by means of a proximal weld 204 at the tapered transition 190 and a distal weld 206 at the leaf tip 198. The wire coil 202 preferably has an outer diameter equal to that of the main wire body 182. By way of example and not limitation, the wire coil 202 may be made of wire having a gauge of about 39 Brown and Sharpe wound with a pitch of about 280 turns per inch. Further, by way of example and not limitation, the leaf 194 may have a thickness from about .001 to about .005 inch and a length of about 0.5 to about 5.0cm. The length of the proximal section 186 may range from about 3 to about 30cm in which case the overall length of the distal portion 184 may range from about 3.5 to about 35 cm. Again, these dimensions are exemplary only; suitable dimensions for a specific case will be evident to those skilled in the art.

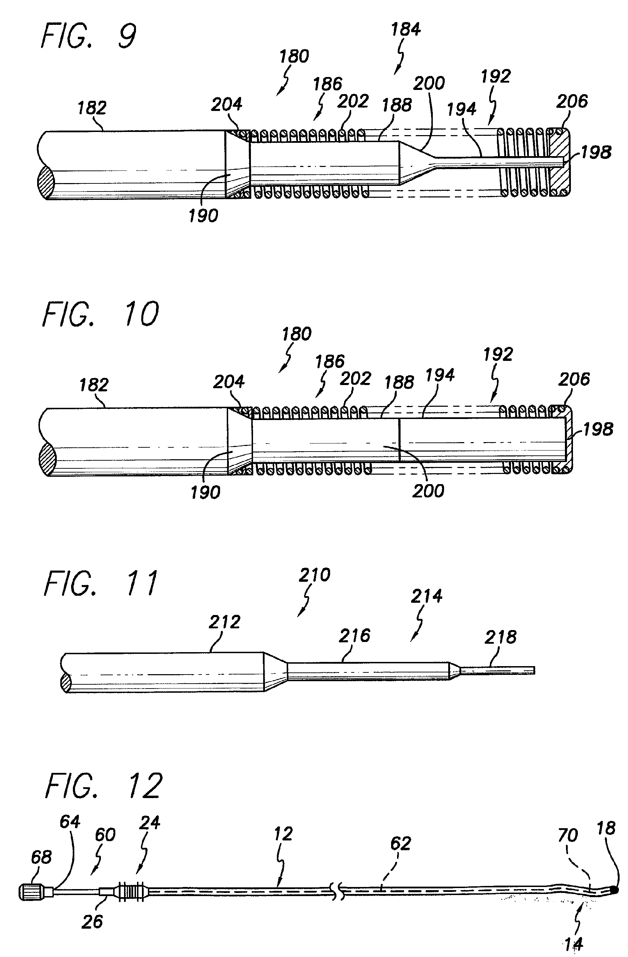

FIG. 11 shows the distal part of a device 210 in accordance with a sixth embodiment of the invention. The device 210 includes a main wire body 212 and a flexible distal portion 214 that, in accordance with a preferred form of the sixth embodiment, comprises a stepped cylindrical structure including a flexible proximal section 216 and an even more flexible distal section 218. The proximal section 216 has a diameter smaller than that of the main wire body 212 and the distal section 218 has a diameter smaller than that of the proximal section 216. The main wire body 212 and the sections 216 and 218 may be fabricated as a one-piece or unitary structure, or may be formed separately and welded or otherwise bonded together. As in the embodiment of FIGS. 8-10, the distal portion 214 may be enveloped within a wire coil welded or otherwise bonded at its proximal and distal ends to the distal portion 214.

Each of the devices 60, 90, 120, 150, 180 and 210 may be coated with PTFE to make it lubricious to facilitate passage through the lumen of the associated lead. A further enhancement may be to coat the device with heparin to inhibit coagulation of the blood on the surface of the device.

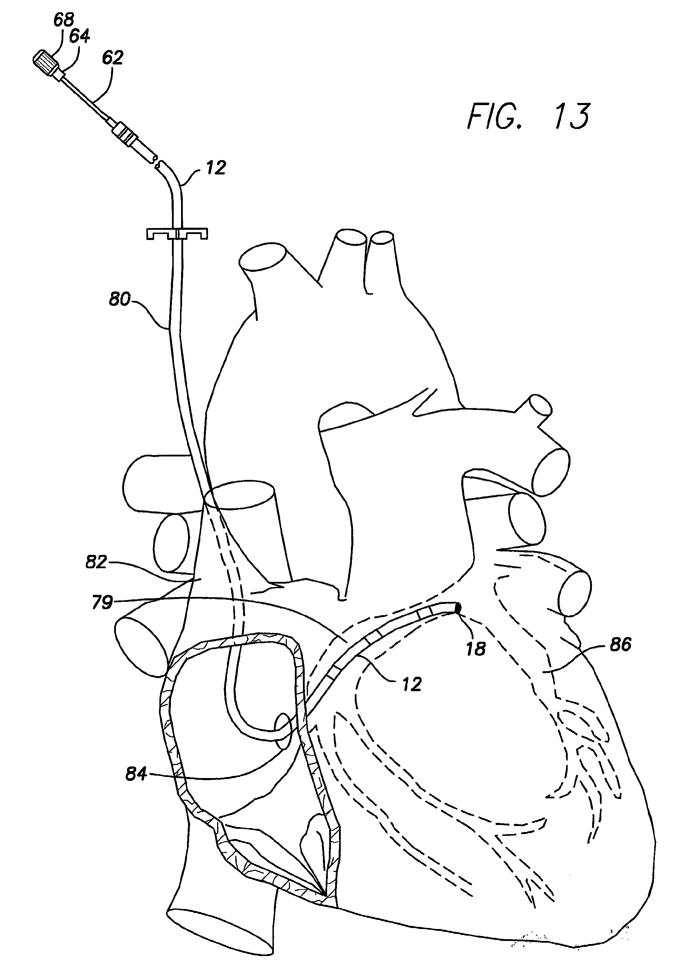

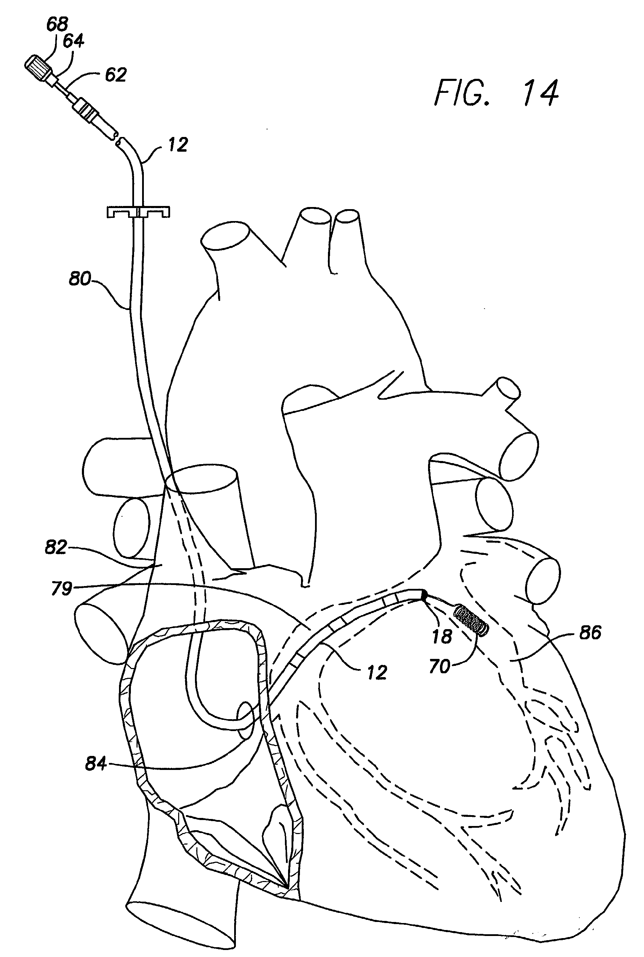

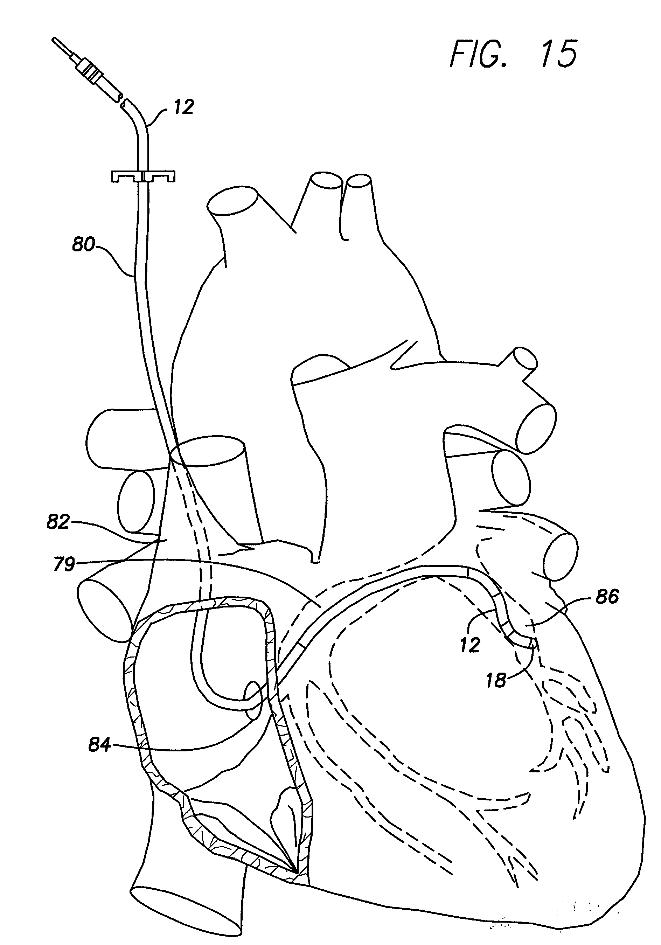

With reference now to FIGS. 12-15, and taking the device 60 as exemplary of the various embodiments of the invention, the steps in using the device of the invention to direct and place a lead body 12 within the coronary sinus region 79 of the heart will now be described. The device 60 is first inserted in the lead body 12 with the flexible distal portion 70 in a retracted position within the distal portion 14 of the lead (FIG. 12). An introducer sheath 80 is then inserted through the superior (SVC) 82 as is well known in the art. The introducer 80 may be long enough to reach the coronary OS 84 and may be precurved to facilitate the directing of the lead. The lead body is inserted into the introducer 80 and advanced therein. (FIG. 13). The device 60 of the invention is then advanced relative to the lead body to extend the flexible distal portion 70 of the device and expose it distally of the tip electrode 18 which defines a central aperture through which the portion 70 passes. (FIG. 14.) By manipulating the steering knob 68 on the proximal extremity 64 of the main wire body 62, the device 60 is maneuvered into position within the coronary sinus region 79 and specifically into the left posterior ventricular (LPV) vein 86 in this particular case. Next, the lead body is moved into place "over-the-wire", that is, it is slid along the device 60 into place within the target coronary vessel. (FIG. 15). Last, the device 60 and the introducer sheath 80 are withdrawn.

It will be seen that unlike the prior art, the lead body 12 provides the guide or delivery tool for the flexible distal portion of the device of the invention. It will also be appreciated that the device of the present invention has the advantage of greatly speeding up the process of lead implantation. It avoids both the relative inflexibility of a stylet and the awkwardness of using an unduly long guide wire yet it is designed for use with a lead body designed for over-the-wire placement with all of the attendant advantages.

## Claims

1. An implantable stimulation lead system, comprising: a lead and a delivery device (60); the lead including a lead body (12) dimensioned for placement inside the coronary sinus region (29), the lead body (12) having at least one electrode (18) positioned at a distal end of the lead body, the distal end of the lead body including a distal tip, the lead further having a lumen extending the length of the lead and communicating with an aperture in the distal tip, the delivery device (60) being dimensioned for insertion within the lumen, the device including a main body (62) and a steering knob (68) secured to a proximal extremity (64) of the main body; **characterised in that** the delivery device (60) includes a flexible distal portion (70) secured to a distal extremity (66) of the main body (60), the main body (60) having a length such that, with the main body (60) of the device substantially completely advanced within the lead, the flexible distal portion (70) of the device projects distally from the aperture in the distal tip of the lead body.

2. A lead system as claimed in Claim 1, **characterised in that** the main body (62) and the flexible distal portion (70) of the device comprise either a unitary structure, or separate structures (92, 100) joined at the distal extremity (96) of the main body (92).

3. A lead system as claimed in Claim 1 or Claim 2, **characterised in that** the main body (62) is formed of wire.

4. A lead system as claimed in Claim 3, **characterised in that** the flexible distal portion (70) of the device comprises a wire coil (72, 102).

5. A lead system as claimed in Claim 4, **characterised in that** the wire coil (72) comprising the flexible distal portion (70) of the device has an outer diameter equal to or smaller than that of the main body (62).

6. A lead system as claimed in any of Claims 3 to 5, **characterised in that** the flexible distal portion (126) of the device comprises a proximal section (128) and a distal section (132), the distal section being more flexible than the proximal section (128).

7. A lead system as claimed in Claim 6, **characterised in that** the proximal section (128) and the distal section (123) of the distal portion (126) of the device comprise wire coils (130, 134).

8. A lead system as claimed in Claim 7, **characterised in that** the wire coil (134) comprising the distal section (132) has an outer diameter smaller than that of the wire coil (130) comprising the proximal section (128).

9. A lead system as claimed in Claim 8, **characterised in that** the wire coil (130) comprising the proximal section (128) has an outer diameter substantially the same as or smaller than that of the main body (122).

10. A lead system as claimed in Claim 6, **characterised in that** the proximal and distal sections (186, 192) comprise a unitary structure.

11. A lead system as claimed in Claim 10, **characterised in that** the proximal and distal sections are cylindrical, the proximal section having an outer diameter smaller than that of the main body and the distal section having an outer diameter smaller than that of the proximal section.

12. A lead system as claimed in Claim 10, **characterised in that** the proximal section (186) is cylindrical (188) and has a diameter smaller than that of the main body (182), and in which the distal section (192) comprises a thin leaf (194).

13. A lead system as claimed in Claim 12, **characterised in that** the thin leaf (194) has a rectangular shape, the leaf (194) having a width equal to the diameter of the proximal section (186).

14. A lead system as claimed in Claim 13, **characterised in that** the flexible distal portion (184) of the device includes a wire coil (202) surrounding the proximal and distal sections (186, 192) of the flexible distal portion (184).

15. A lead system as claimed in Claim 14, **characterised in that** the thin leaf (194) includes a distal tip (198), the wire coil (202) surrounding the proximal and distal sections (186, 192) of the flexible distal portion (184) having an end attached to the distal tip (198) of the thin leaf (194) and another end attached to the distal extremity of the main body (182).

16. A device (160) for delivering a body implantable lead, the device comprising a main wire body (62) having a proximal extremity and a distal extremity and a steering knob (68) secured to the proximal extremity (64) of the main wire body (62), **characterised in that** the device includes a flexible distal portion (70) having a proximal end secured to the distal extremity (66) of the main body, the flexible distal portion (70) comprising a wire coil (72, 102).

17. A device as claimed in Claim 16, **characterised in that** the wire coil (70,102) comprising the flexible distal portion (70) of the device has an outer diameter either substantially the same as or smaller than that of the main body.

18. A device as claimed in Claim 16, **characterised in that** the wire coil comprising the flexible distal portion (126, 154) of the device includes a proximal section (128, 156) and a distal section (132, 158), the distal section being more flexible than the proximal section.

19. A device as claimed in Claim 18, **characterised in that** the distal section (1132, 158) of the wire coil has an outer diameter smaller than that of the proximal section (128, 156) of the wire coil.

20. A device as claimed in Claim 19, **characterised in that** the proximal section (128) of the wire coil has an outer diameter substantially equal to or smaller than that of the main body (122).

21. A device for delivering a body implantable lead, the device comprising a main wire body (122) having a proximal extremity and a distal extremity and a steering knob (68) secured to the proximal extremity of the main wire body (122), **characterised in that** the device includes a flexible distal portion (126) having a proximal end secured to the distal extremity of the main body (62), the flexible distal portion (126) of the device comprising a proximal section (128) and a distal section (132), the distal section (132) being more flexible than the proximal section (128).

22. A device as claimed in Claim 21, **characterised in that** the proximal section (128) and the distal section (132) of the flexible distal portion (126) of the device comprise wire coils.

23. A device as claimed in Claim 22, **characterised in that** the wire coil comprising the distal section (132) has an outer diameter smaller than that of the wire coil comprising the proximal section (128).

24. A device as claimed in Claim 23, **characterised in that** the wire coil comprising the proximal section (128) has an outer diameter substantially the same as or smaller than that of the main body (122).

25. A device as claimed in Claim 21, **characterised in that** the proximal and distal sections comprise a unitary structure.

26. A device as claimed in Claim 25, **characterised in that** the proximal and distal sections (216, 218) are cylindrical, the proximal section (216) having an outer diameter smaller than that of the main body (212) and the distal section (218) having an outer diameter smaller than that of the proximal section (216).

27. A device as claimed in Claim 25, **characterised in that** the proximal section (186) is cylindrical and has a diameter smaller than that of the main body (182), and in which the distal section (192) comprises a thin leaf (194).

28. A device as claimed in Claim 27, **characterised in that** the thin leaf (194) has a rectangular shape with a width substantially the same as the diameter of the proximal section (186).

29. A device as claimed in Claim 28, **characterised in that** the flexible distal portion (184) of the device includes a wire coil (202) surrounding the proximal and distal sections (186, 192) of the flexible distal portion (184).

30. A device as claimed in Claim 29, **characterised in that** the thin leaf (194) includes a distal tip (198), the wire coil surrounding the proximal and distal sections of the flexible distal portion having one end attached to the distal tip of the thin leaf (194) and another end attached to the proximal end of the flexible distal portion (184).
